# EUROPEAN PATENT SPECIFICATION

(11) **EP 1 000 013 B1**
(45) Date of publication and mention of the grant of the patent: **15.05.2002**
(21) Application number: 98932480.1
(22) Date of filing: 26.06.1998
(51) Int. Cl.: C07C 229/00

(54) **PROCESS FOR ISOMERISATION OF TILIDINE**
VERFAHREN ZUR ISOMERISIERUNG VON TILIDIN
PROCEDE POUR L'ISOMERISATION DE LA TILIDINE

(30) Priority: 14.07.1997 IE 970517; 25.07.1997 IE 970542
(43) Date of publication of application: 17.05.2000
(73) Proprietor: RUSSINSKY LIMITED, Cork (IE)
(72) Inventor: NIKOLOPOULOS, Angelo, Cork (IE); SCHICKANEDER, Helmut, Cork (IE); MULCAHY, David, Cork (IE)
(74) Representative: O'Brien, John Augustine
(86) International application number: IE9800052
(87) International publication number: WO9903821

(56) References cited:
- DE-B- 1 518 959
- US-A- 3 557 127
- US-A- 3 679 732

## Description

### Introduction

The invention relates to a process for isomerisation of Tilidine and derivatives thereof.

Tilidine hydrochloride is a potent therapeutically active analgesic compound which has been used successfully for the treatment of intense and strong pain. It is described in US 3,557,127, DE-C-1518959 and DE-C-1793571.

The compound is a substituted cyclohexanone derivative having the INN chemical name ±-Ethyl-(trans-2-dimethylamine-1-phenyl-3-cyclohexen-trans-1-carboxylate).

Tilidine can exist as either the trans or cis isomer. In this specification trans-tilidine means the racemic mixture of trans-tilidine as shown by the following chemical structures.

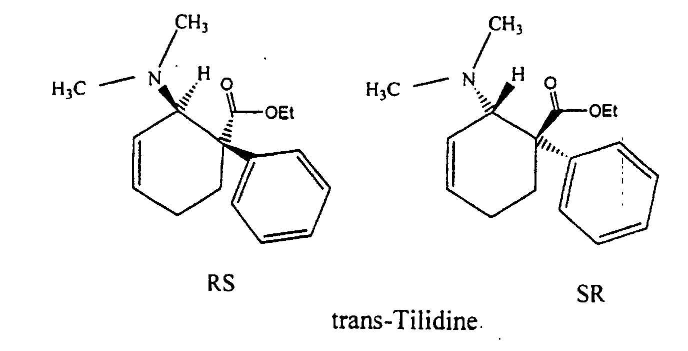

It has been shown (Liebigs Ann. Chem. 728, 64-87 [1969]) that the reaction of dimethylamine-1, 3-butadiene with ethylatropate leads to a cis/trans mixture of Tilidine in a ratio of approximately 80:20.

It is known (Liebigs Ann. Chem. 728, 64-87 [1969]) and accepted that the stereospecific character of Diene (1,4) - Cyclo addition is dominated by a relative reciprocal orientation of the Ene-and Diene components before the reaction. This means the constituents of the components introduce specific amounts of electronic density during the reaction. Furthermore, the orientation of the constituents in the cyclohexene ring is influenced also by that steric factor. Consequently, the cis/trans position in the ring will be directed by their "bulky" constituents.

US 3,557,127 describes methods to rearrange or to separate the cis-isomer to produce the trans-isomer of cyclohexenes. However, such methods are inefficient and uneconomic, especially at a commercial production scale.

US 3 ,679,732 describes a process for partial isomerisation of the cis-isomer in the presence of organic acids such as aliphatic or aromatic monocarboxylic acids, aliphatic or aromatic dicarboxylic acids or anhydrides. The conversion of cis-Tilidine into trans-Tilidine is relatively low and salt impurities are produced.

There is therefore a need for an improved commercially viable process in which the yield of the trans-isomer is maximised and the amount of the cis-isomer present is minimised.

This invention is directed towards providing such a process.

### Statements of Invention

According to the invention, there is provided a process for preparing Tilidine comprising:-
mixing a substantially pure cis Tilidine base with water and an inorganic acid at pH values between 2 and 6; and
heating the reaction mixture thus formed to yield isomerised cis/trans Tilidine base with an enhanced content of the trans isomer.

Preferably, the pH is from 3 to 6.

Most preferably the acid is phosphoric acid. This is particularly important as it provides high yield and conversion rate.

Particularly preferred because of improved yield and conversion rate are processes in which the acid, especially phosphoric acid, is added in an amount of from 0.5 to 2.0, most preferably 0.9 to 1.1, and ideally approximately 1:1 molar equivalent to the Tilidine base.

Ideally, the reaction mixture is heated to a temperature of from 90°C to 100°C and kept at this temperature for from 15 to 30 hours.

In a particularly preferred aspect, the invention provides a process comprising mixing cis/trans Tilidine base with water and phosphoric acid, and heating the reaction mixture thus formed to yield isomerised cis/trans Tilidine base with an enhanced content of the trans isomer.

Surprisingly we found that, heating under specific conditions with an acid especially ortho phosphoric acid at pH-values between 2 - 6 (especially 3 - 6) in water as a solvent at 50 - 100°C (especially 95 - 100°C) conversion (cis to trans isomerisation) is achieved in high yields, substantially without side products/impurities.

Preferably, the isomerised Tilidine base has a content of greater than 10% of the trans isomer.

In a preferred embodiment of the invention, isomerised Tilidine base has a content of greater than 60% of the trans isomer.

In one embodiment of the invention the acid comprises a mixture of phosphoric acid and salts thereof.

In one embodiment of the invention, after heating, the reaction mixture is cooled and the cis/trans Tilidine base is extracted by liquid-liquid extraction. Preferably, a solvent such as petroleum ether is added to the solution, the pH is adjusted with a base to 12 - 14, the aqueous layer is separated and the organic layer is evaporated to give the isomerised cis/trans Tilidine base.

### Detailed Description of the Invention

The invention will be more clearly understood from the following description thereof, given by way of example only.

### General Isomerisation Procedure

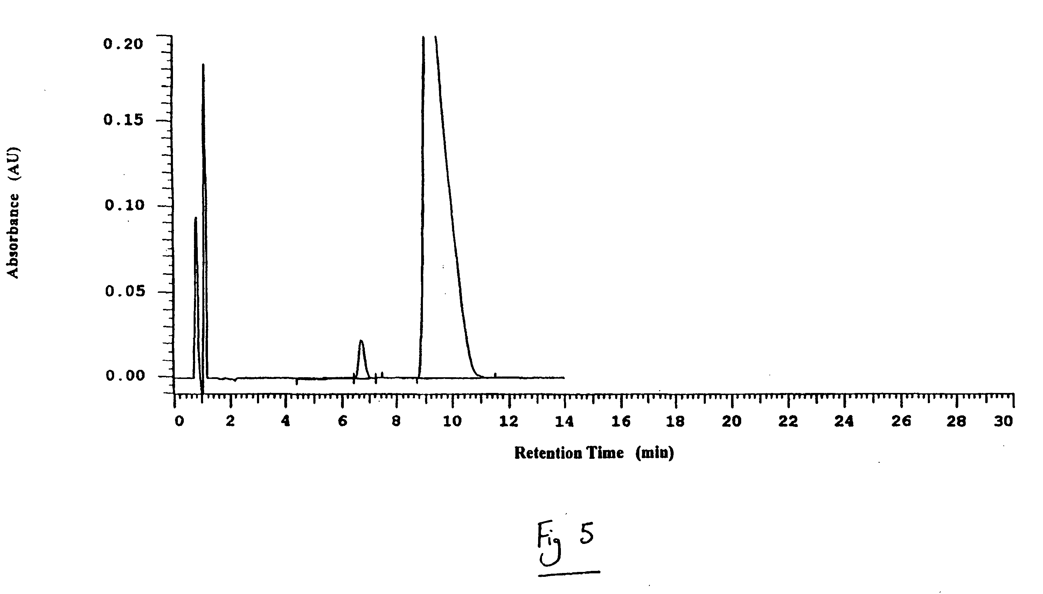

10 g of cis-Tilidine base (36.58 mmole ≥ 97% cis pure by HPLC analysis as shown in Fig. 5) are mixed with 30 mls of process water and 4.3 g of phosphoric acid (85%) (37.3mmol). The reaction mixture is heated to reflux at 95 to 100°C and kept for 15 to 30 hours. The solution is then cooled to 40 - 50°C, an extra 30mls of process water are added together with 50 to 60 mls of petroleum ether. The pH is adjusted to 12 - 14 with sodium hydroxide (30%) and the aqueous layer is separated at 40 - 50°C. After washing the organic layer with 20 to 30 mls of process water, the organic layer is evaporated to dryness to give isomerised (cis/trans) Tilidine base.

The yield is 6 to 8 g Tilidine base (yellow oil) with enriched trans-Tilidine.

### Example 1

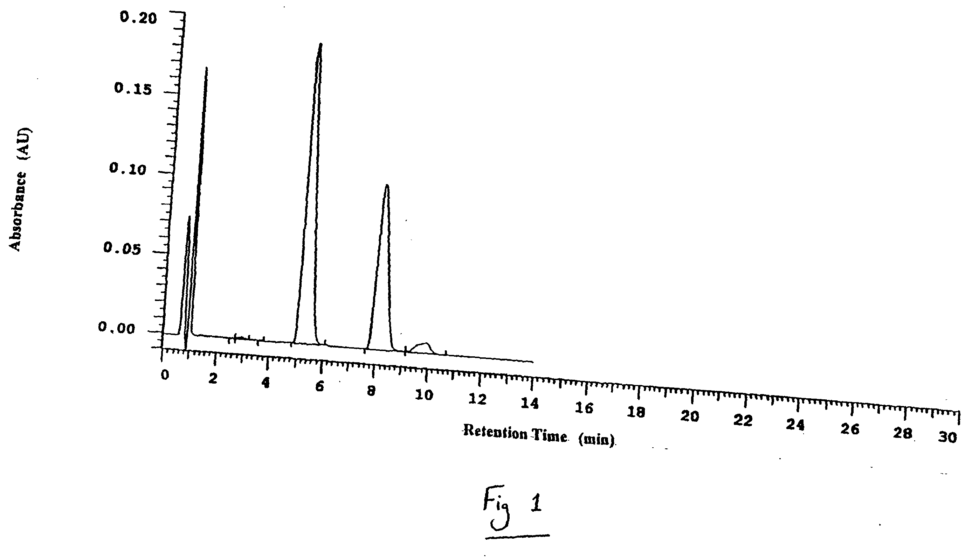

Following the general procedure outlined above, 10 g of pure cis Tilidine base was heated with 0.8 molar amount of phosphoric acid (85%) at pH 5 - 6 in process water at 95 to 100°C for 16 hours to yield 8.8 g of a mixture of 39.5 : 56.8% of cis/trans Tilidine base. The HPLC analysis is given in Fig. 1.

Thus, a high yield and an excellent conversion rate are achieved.

### Example 2

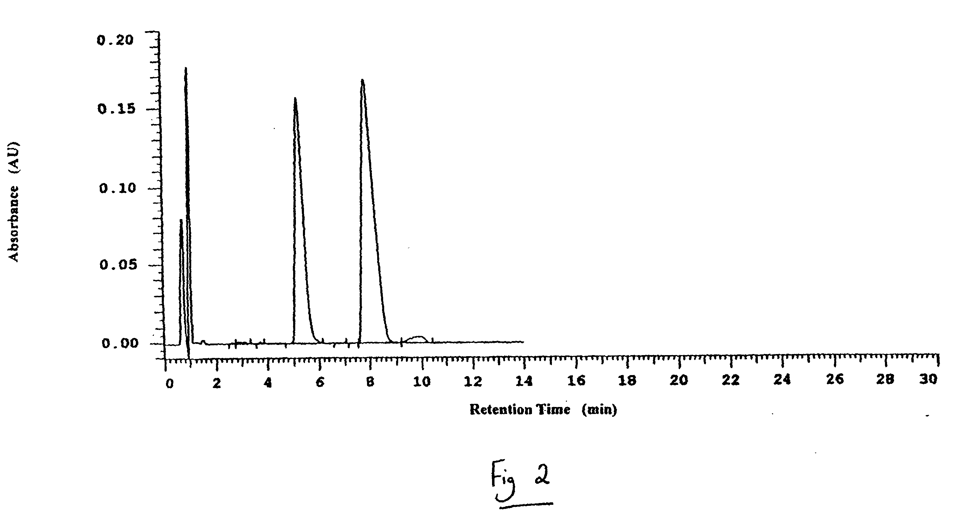

Following the general procedure outlined above, 10 g of pure cis Tilidine base was heated with 0.5 molar amount of phosphoric acid (85%) at pH 5 - 6 in process water at 95 to 100°C for 20.5 hours to yield 7.5 g of a mixture of 60.1:37.8% of cis/trans Tilidine base. The HPLC analysis is given in Fig. 2.

### Example 3

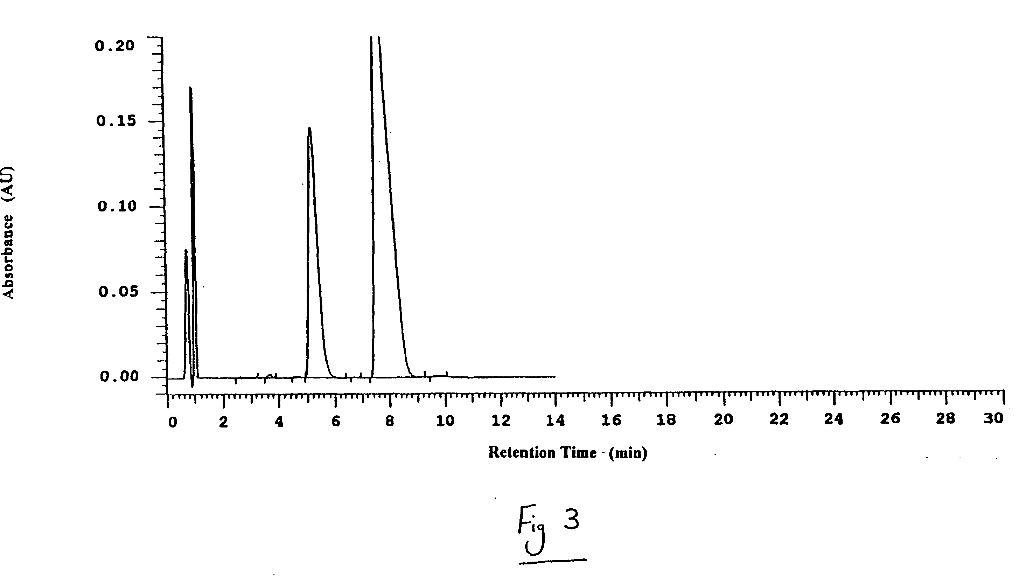

Following the general procedure outlined above, 10 g of pure cis Tilidine base was heated with 2 molar amount of phosphoric acid (85%) at pH 2 - 3 in process water at 95 to 100°C for 16 hours to yield 7.9 g of a mixture of 73.8:25.7% of cis/trans Tilidine base. The HPLC analysis is given in Fig. 3.

### Example 4

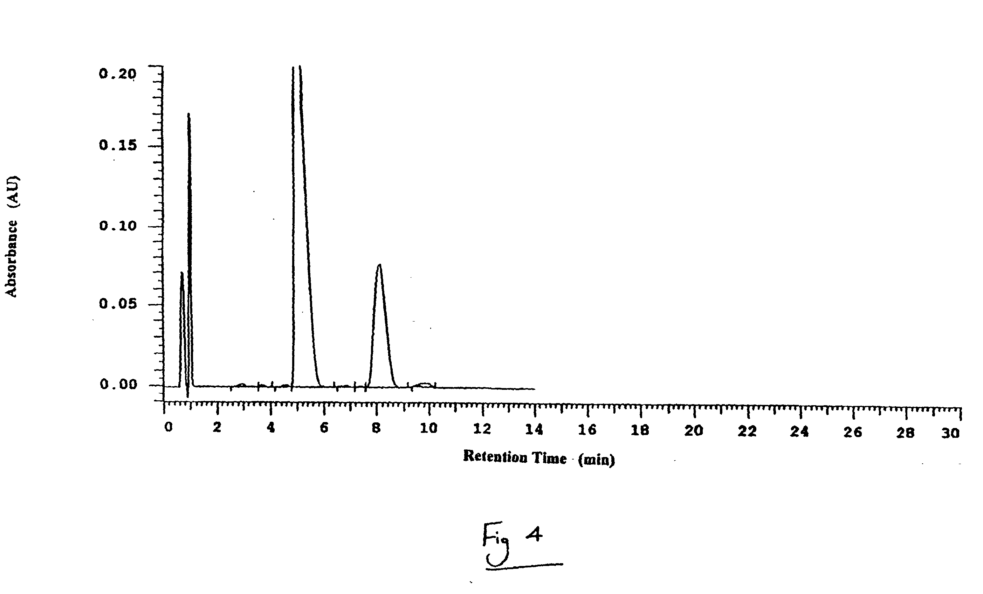

Following the general procedure outlined above, 10 g of pure cis Tilidine base was heated with an equimolar amount of phosphoric acid (85%) at pH 3 - 4 in process water at 95 to 100°C for 30 hours to yield 6.4 g of a mixture of 25.1:73.2% of cis/trans Tilidine base. The HPLC analysis is given in Fig. 4.

### Example 5

Following the general procedure outlined above, 10 g of pure cis Tilidine base was heated with an equimolar amount of phosphoric acid (85%) at pH 3 - 4 in process water at 95 to 100°C for 7.5 hours to yield 8.5 g of a mixture of 69:31% cis/tans Tilidine base as determined by HPLC analysis.

### Example 6

Following the general procedure outlined above, 10 g of pure cis Tilidine base was heated with an equimolar amount of phosphoric acid (85%) at pH 3 - 4 in process water at 95 to 100°C for 18.5 hours to yield 7.0 g of a mixture of 34.3:61.7% of cis/trans Tilidine base as determined by HPLC analysis.

### Example 7

Following the general procedure outlined above, 10 g of pure cis Tilidine base was heated with 1.2 molar amount of phosphoric acid (85%) at pH 3 in process water at 95 to 100°C for 15.5 hours to yield 7.5 g of a mixture of 42.5:54.7% of cis/trans Tilidine base as determined by HPLC analysis.

### Example 8

Following the general procedure outlined above, 10.8 g of pure cis Tilidine base was heated with an equimolar amount of phosphoric acid (85%) at pH 3 - 4 in process water at 78 to 80°C for 17 hours to yield 9.4 g of a mixture of 86.2:13.5% of cis/trans Tilidine base as determined by HPLC analysis.
The lower reaction temperature leads to a lower conversion.

### Example 9

Following the general procedure outlined above, 6.2 g of pure cis Tilidine HCl. 1.5 H₂O was heated at pH 5 - 6 in process water at 95 to 100°C for 20 hours to yield 4.6 g of a mixture of 90.9:8.8% cis/trans Tilidine base as determined by HPLC analysis.
The use of the Tilidine Hydrochloride salt gives less than 10% converion.

### Example 10

Following the general procedure outlined above, 10 g of pure cis Tilidine base was heated with an equimolar amount of concentrated H₂SO₄ at pH 1 in process water at 95 to 100°C for more than 16 hours to yield 8.5 g of a mixture of 94.1:4.9% of cis/trans Tilidine base as determined by HPLC.

### Example 11

Following the general procedure outlined above, 10 g of pure cis Tilidine base was heated with an equimolar amount of KH₂PO₄ at pH 5 in process water at 95 to 100°C for 21 hours to yield 8.5 g of a mixture of 91.4:8.6% cis/trans Tilidine base as determined by HPLC.

### Example 12

Following the general procedure outlined above, 10.1 g of pure cis Tilidine base was heated with an equimolar amount of NaH₂PO₄ at pH 5 - 6 in process water at 95 to 100°C for 18.5 hours to yield 8.6 g of a mixture of 88.9:10.8% cis/trans Tilidine base as determined by HPLC.

We have surprisingly found that the best results in terms of yield and conversion rate with optimum reaction conditions are achieved with 0.5 to 2.0, especially 0.9 to 1.1, ideally approximately 1:1 molar equivalents of phosphoric acid. We have found that other acids provide enhanced yield and/or conversion rates and/or more favourable reaction conditions than prior art processes.

### APPENDIX

### Data for Fig. 1

| Peak Quantitation: AREA | | | Calculation Method: AREA% | | |
|---|---|---|---|---|---|
| No. | RT | Area | Area% | BC | Purity |
| 1 | 2.61 | 1987 | 0.058 | BB | 0.475 |
| 2 | 2.98 | 10185 | 0.298 | BB | 0.899 |
| 3 | 3.69 | 2021 | 0.059 | BB | 0.671 |
| 4 | 5.16 | 1938626 | 56.652 | BB | 1.000 |
| 5 | 8.03 | 1346259 | 39.341 | BB | 0.999 |
| 6 | 9.89 | 122924 | 3.592 | BB | 0.996 |
| | | 3422002 | 100.000 | | |

### Data for Fig. 2

| Peak Quantitation: AREA | | | Calculation Method: AREA% | | |
|---|---|---|---|---|---|
| No. | RT | Area | Area% | BC | Purity |
| 1 | 2.63 | 1879 | 0.045 | BB | 0.974 |
| 2 | 2.87 | 12516 | 0.300 | BB | 0.888 |
| 3 | 3.71 | 4196 | 0.100 | BB | 0.993 |
| 4 | 5.22 | 1576846 | 37.751 | BB | 1.000 |
| 5 | 6.83 | 2350 | 0.056 | BB | 0.991 |
| 6 | 7.32 | 1636 | 0.039 | BB | 0.732 |
| 7 | 7.85 | 2503892 | 59.945 | BV | 0.999 |
| 8 | 9.90 | 73673 | 1.764 | TBB | 0.996 |
| | | 4176988 | 100.000 | | |

### Data for Fig. 3

| Peak Quantitation: AREA | | | Calculation Method: AREA% | | |
|---|---|---|---|---|---|
| No. | RT | Area | Area% | BC | Purity |
| 1 | 2.61 | 7456 | 0.131 | BB | 0.841 |
| 2 | 3.69 | 7850 | 0.138 | BB | 0.997 |
| 3 | 4.71 | 3251 | 0.057 | BB | 0.898 |
| 4 | 5.21 | 1463439 | 25.769 | BB | 1.000 |
| 5 | 6.75 | 1885 | 0.033 | BB | 0.968 |
| 6 | 7.62 | 4191946 | 73.814 | BB | 1.000 |
| 7 | 9.89 | 3232 | 0.057 | BB | 0.690 |
| | | 5679059 | 100.000 | | |

### Data for Fig. 4

| Peak Quantitation: AREA | | | Calculation Method: AREA% | | |
|---|---|---|---|---|---|
| No. | RT | Area | Area% | BC | Purity |
| 1 | 2.97 | 15142 | 0.357 | BB | 0.989 |
| 2 | 3.69 | 5455 | 0.129 | BB | 0.842 |
| 3 | 4.61 | 7039 | 0.166 | BB | 0.960 |
| 4 | 5.03 | 3101901 | 73.133 | BB | 1.000 |
| 5 | 6.83 | 7642 | 0.180 | BB | 0.936 |
| 6 | 7.36 | 4447 | 0.105 | BB | 0.779 |
| 7 | 8.11 | 1064949 | 25.108 | BB | 1.000 |
| 8 | 9.91 | 34882 | 0.822 | BB | 0.990 |
| | | 4241457 | 100.000 | | |

### Data for Fig. 5

| Peak Quantitation: AREA | | | Calculation Method: AREA% | | |
|---|---|---|---|---|---|
| No. | RT | Area | Area% | BC | Purity |
| 1 | 6.27 | 22677 | 0.327 | BB | 0.982 |
| 2 | 6.71 | 148290 | 2.138 | BB | 1.000 |
| 3 | 7.41 | 1259 | 0.018 | BB | 0.970 |
| 4 | 9.13 | 6764288 | 97.517 | BB | 1.000 |
| | | 6936514 | 100.000 | | |

## Claims

1. A process for preparing Tilidine comprising: -
mixing a substantially pure cis Tilidine base with water and an inorganic acid at pH values between 2 and 6; and
heating the reaction mixture thus formed to yield isomerised cis/trans Tilidine base with an enhanced content of the trans isomer.

2. A process as claimed in claim 1, wherein the pH is from 3 to 6.

3. A process as claimed in claim 1 or 2 wherein the acid is phosphoric acid.

4. A process as claimed in any of claims 1 to 3 wherein the acid is added in from 0.5 to 2.0 molar equivalents of the Tilidine base.

5. A process as claimed in claim 4 wherein the acid is added in from 0.9 to 1.1 molar equivalents of the Tilidine base.

6. A process as claimed in claim 4 or 5 wherein the acid is added in approximately equimolar equivalent to Tilidine base.

7. A process as claimed in any preceding claim wherein the reaction mixture is heated to a temperature of from 90°C to 100°C and kept at this temperature for a period of from 15 to 30 hours.

8. A process for preparing Tilidine comprising mixing cis/trans Tilidine base with water and phosphoric acid, and heating the reaction mixture thus formed to yield isomerised cis/trans Tilidine base with an enhanced content of the trans isomer.

9. A process as claimed in any preceding claim, wherein isomerised Tilidine base has a content of greater than 10% of the trans isomer.

10. A process as claimed in claim 9 wherein isomerised Tilidine base has a content of greater than 60% of the trans isomer.

11. A process as claimed in any preceding claim, wherein the acid comprises a mixture of phosphoric acid and salts thereof.

12. A process as claimed in any preceding claim, wherein after heating, the reaction mixture is cooled and the cis/trans Tilidine base is extracted by liquid-liquid extraction.

13. A process as claimed in claim 12, wherein a solvent such as petroleum ether is added to the solution, the pH is adjusted with a base to 12 - 14, the aqueous layer is separated and the organic layer is evaporated to give the isomerised cis/trans Tilidine base.

## Patentansprüche

1. Verfahren zur Herstellung von Tilidin, umfassend:
- Mischen einer im wesentlichen reinen *cis*-Tilidinbase mit Wasser und einer anorganischen Säure bei einem pH-Wert zwischen 2 und 6; und
- Erhitzen des auf diese Weise gebildeten Reaktionsgemisches, um eine isomerisierte *cis*/*trans*-Tilidinbase mit einem verbesserten Gehalt des *trans-*Isomers zu gewinnen.

2. Verfahren nach Anspruch 1, worin der pH-Wert von 3 bis 6 ist.

3. Verfahren nach Anspruch 1 oder 2, worin die Säure Phosphorsäure ist.

4. Verfahren nach einem der Ansprüche 1 bis 3, worin die Säure in einer Menge von 0,5 bis 2,0 Moläquivalenten, bezogen auf die Tilidinbase, zugegeben wird.

5. Verfahren nach Anspruch 4, worin die Säure in einer Menge von 0,9 bis 1,1 Moläquivalenten, bezogen auf die Tilidinbase, zugegeben wird.

6. Verfahren nach Anspruch 4 oder 5, worin die Säure etwa in äquimolarer Menge zu der Tilidinbase zugegeben wird.

7. Verfahren nach einem der vorstehenden Ansprüche, worin das Reaktionsgemisch auf eine Temperatur von 90°C bis 100°C erwärmt wird und bei dieser Temperatur für einen Zeitraum von 15 bis 30 Stunden gehalten wird.

8. Verfahren zur Herstellung von Tilidin, umfassend das Mischen einer *cis*/*trans*-Tilidinbase mit Wasser und Phosphorsäure und Erwärmen des auf diese Weise gebildeten Reaktionsgemischs, um isomerisierte *cis*/*trans*-Tilidinbase mit einem verbesserten Gehalt des *trans*-Isomers zu gewinnen.

9. Verfahren nach einem der vorstehenden Ansprüche, worin die isomerisierte Tilidinbase einen Gehalt von über 10 % des *trans*-Isomers aufweist.

10. Verfahren nach Anspruch 9, worin die isomerisierte Tilidinbase einen Gehalt von über 60 % des *trans*-Isomers aufweist.

11. Verfahren nach einem der vorstehenden Ansprüche, worin die Säure ein Gemisch aus Phosphorsäure und Salzen davon umfasst.

12. Verfahren nach einem der vorstehenden Ansprüche, worin das Reaktionsgemisch nach dem Erwärmen abgekühlt und die *cis*/*trans*-Tilidinbase durch Flüssig-Flüssig-Extraktion extrahiert wird.

13. Verfahren nach Anspruch 12, worin ein Lösungsmittel wie Petrolether zu der Lösung zugegeben wird, der pH-Wert mit einer Base auf 12 bis 14 eingestellt wird, die flüssige Schicht abgetrennt und die organische Schicht verdampft wird, so dass die isomerisierte *cis*/*trans*-Tilidinbase entsteht.

## Revendications

1. Procédé de préparation de Tilidine, comprenant :
le mélange d'une base de cis-Tilidine essentiellement pure avec de l'eau et un acide inorganique à des valeurs de pH comprises entre 2 et 6 ; et
le chauffage du mélange réactionnel ainsi formé pour conduire à une base de cis/trans-Tilidine isomérisée ayant une teneur en isomère trans améliorée.

2. Procédé selon la revendication 1, dans lequel le pH va de 3 à 6.

3. Procédé selon la revendication 1 ou 2, dans lequel l'acide est l'acide phosphorique.

4. Procédé selon l'une quelconque des revendications 1 à 3, dans lequel l'acide est ajouté en une quantité de 0,5 à 2,0 équivalents molaires par rapport à la base de Tilidine.

5. Procédé selon la revendication 4, dans lequel l'acide est ajouté en une quantité de 0,9 à 1,1 équivalents molaires par rapport à la base de Tilidine.

6. Procédé selon la revendication 4 ou 5, dans lequel l'acide est ajouté en une quantité approximativement équimolaire en équivalents par rapport à la base de Tilidine.

7. Procédé selon l'une quelconque des revendications précédentes, dans lequel le mélange réactionnel est chauffé jusqu'à une température de 90°C à 100°C et maintenu à cette température pour une durée de 15 à 30 heures.

8. Procédé de préparation de Tilidine, comprenant le mélange d'une base de cis/trans-Tilidine avec de l'eau et de l'acide phosphorique, et le chauffage du mélange réactionnel ainsi formé pour conduire à une base de cis/trans-Tilidine isomérisée ayant une teneur en isomère trans améliorée.

9. Procédé selon l'une quelconque des revendications précédentes, dans lequel la base de Tilidine isomérisée a une teneur supérieure à 10% en isomère trans.

10. Procédé selon la revendication 9, dans lequel la base de Tilidine isomérisée a une teneur supérieure à 60% en isomère trans.

11. Procédé selon l'une quelconque des revendications précédentes, dans lequel l'acide comprend un mélange d'acide phosphorique et de sels de celui-ci.

12. Procédé selon l'une quelconque des revendications précédentes, dans lequel après chauffage, le mélange réactionnel est refroidi et la base de cis/trans-Tilidine est extraite par une extraction liquide/liquide.

13. Procédé selon la revendication 12, dans lequel un solvant tel que l'éther de pétrole est ajouté à la solution, le pH est ajusté jusqu'à 12-14 par une base, la couche aqueuse est séparée et la couche organique est évaporée pour conduire à la base de cis/trans-Tilidine isomérisée.
